(19) Europäisches Patentamt
European Patent Office
Office européen des brevets

(11) Numéro de publication : **0 389 337 B1**

# (12) FASCICULE DE BREVET EUROPEEN

(45) Date de publication du fascicule du brevet : **10.02.93 Bulletin 93/06**

(51) Int. Cl.$^5$ : **A61K 7/06,** A61K 7/42, C08G 77/38

(21) Numéro de dépôt : **90400702.8**

(22) Date de dépôt : **16.03.90**

## (54) Utilisation en cosmétique de diorganopolysiloxanes à fonction hydroxy-2 benzophénone et compositions contenant ces composés, destinées à la protection de la peau et des cheveux.

(30) Priorité : **22.03.89 FR 8903783**

(43) Date de publication de la demande : **26.09.90 Bulletin 90/39**

(45) Mention de la délivrance du brevet : **10.02.93 Bulletin 93/06**

(84) Etats contractants désignés : **AT BE CH DE DK ES FR GB GR IT LI NL SE**

(56) Documents cités :
**EP-A- 0 138 590**
**FR-A- 1 518 231**
**US-A- 2 938 047**
**CHEMICAL ABSTRACTS, vol. 103, no. 22, décembre 1985, page 343, résumé no. 183382p, Columbus, Ohio, US; & JP-A-60 99 186(POLA CHEMICAL INDUSTRIES, LTD) 03-06-1985**

(56) Documents cités :
**DIE MAKROMOLEKULARE CHEMIE, vol. 188, no. 11, novembre 1987, pages 2759-2767, Basel, CH; G. NESTOR et al.: "Purity ofliquid-crystal polysiloxanes, 1 - Isolation procedures"**

(73) Titulaire : **L'OREAL**
**14, Rue Royale**
**F-75008 Paris (FR)**

(72) Inventeur : **Forestier, Serge**
**16 allée Ferdinand Buisson**
**F-77410 Claye-Souilly (FR)**
Inventeur : **Lang, Gérard**
**44, avenue Lacour**
**F-95210 Saint-Gratien (FR)**
Inventeur : **Richard, Hervé**
**48, rue de l'Ermitage**
**F-75020 Paris (FR)**

(74) Mandataire : **Casalonga, Axel et al**
**BUREAU D.A. CASALONGA - JOSSE**
**Morassistrasse 8**
**W-8000 München 5 (DE)**

**EP 0 389 337 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à l'utilisation en cosmétique, notamment en tant qu'agents filtrant le rayonnement UV, de diorganopolysiloxanes à fonction hydroxy-2 benzophénone ainsi qu'aux nouvelles compositions cosmétiques contenant ces composés, destinées à la protection de la peau et des cheveux.

On sait que les radiations lumineuses de longueurs d'onde comprises entre 280 nm et 400 nm permettent le brunissement de l'épiderme humain et que les rayons de longueurs d'onde comprises entre 280 et 320 nm, connues sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il est donc intéressant de disposer de composés absorbant les rayons UV sur une large bande afin de pouvoir filtrer à la fois les rayons UV-A et UV-B.

On sait par ailleurs que la lumière agresse la kératine des cheveux. De nombreuses publications divulguent que la lumière naturelle détruit certains aminoacides des cheveux et qu'en altérant la fibre capillaire, elle en diminue les propriétés mécaniques; cette diminution des propriétés mécaniques peut être mise en évidence par la diminution du palier à 15% d'extension sur cheveu mouillé ou par l'augmentation de la solubilité alcaline.

Le palier à 15% d'extension est le poids qu'il faut appliquer à un cheveu mouillé d'une longueur donnée pour l'allonger de 15%. Plus le poids est élevé, plus le cheveu est élastique et résistant.

La solubilité alcaline permet d'évaluer la dégradation des chaînes polypeptidiques du cheveu par dosage de la matière protéique solubilisée dans une solution alcaline.

Plus la solubilité alcaline est élevée, plus le cheveu est altéré et dégradé.

Il est donc souhaitable d'assurer aux cheveux une bonne protection contre la dégradation photochimique afin de préserver leurs propriétés mécaniques et éviter leur décoloration ou changement de nuance.

On sait aussi que les constituants entrant dans les préparations cosmétiques ne possèdent pas toujours une stabilité suffisante à la lumière et qu'ils se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement utilisés en cosmétique, et en particulier dans les huiles et graisses.

On sait, par ailleurs, greffer sur des chaînes de polymères carbonés synthétiques, sur des polymères naturels, comme les protéines ou les hydrolysats de protéines ou encore sur des polyaminoamides, des restes de molécules ayant un effet filtre vis-à-vis du rayonnement UV; ces polymères greffés décrits par exemple dans les brevets français n° 2 197 023, 2 237 912, 2 531 960, 2 548 018, 2 549 069, 2 586 692 et 2 586 693 peuvent être utilisés pour préparer des compositions cosmétiques protectrices de l'épiderme humain ou anti-solaires. On a cependant constaté que ces polymères greffés sont généralement peu solubles dans les solvants cosmétiques usuels, notamment dans les supports gras, et qu'ils forment des films dont la structure est trop rigide.

Or, la demanderesse a découvert que certains diorganopolysiloxanes à fonction hydroxy-2 benzophénone présentaient, de manière étonnante, de bonnes propriétés cosmétiques associées à de bonnes propriétés filtrantes dans une large gamme de longueurs d'onde allant de 280 à 360 nm. Ils présentent notamment un excellent caractère liposoluble, ce qui les rend utilisables dans les supports gras utilisés en cosmétique. Outre leur bon pouvoir filtrant et leur bonne solubilité dans les corps gras et les solvants cosmétiques usuels, ces diorganopolysiloxanes à fonction hydroxy-2 benzophénone présentent une excellente stabilité chimique et photochimique et sont bien tolérés par la peau et les cheveux auxquels ils apportent de la douceur.

Ils ont en outre l'avantage de préserver les propriétés mécaniques et la couleur des cheveux de la dégradation par la lumière. Cet avantage a pu être mis en évidence par exposition en lumière naturelle (milieu ensoleillé) et en lumière artificielle (émetteur au xénon d'un appareil de vieillissement accéléré du type SUNTEST HANAU).

La présente invention a donc pour objet l'utilisation en cosmétique, en tant qu'agents filtrant le rayonnement UV de longueurs d'onde comprises entre 280 et 360 nm, de diorganopolysiloxanes à fonction hydroxy-2 benzophénone choisis parmi ceux de formule :

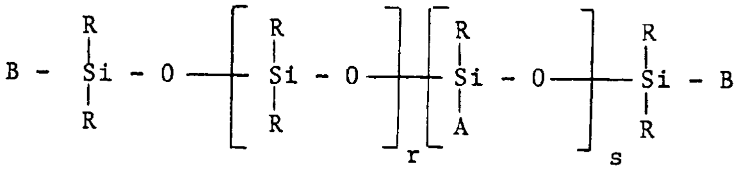 (1)

dans laquelle les symboles :

R, identiques ou différents, sont choisis parmi les radicaux alkyle linéaires ou ramifiés en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

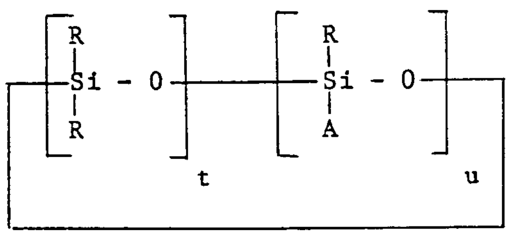 (2)

dans laquelle

R a la même signification qu'à la formule (1),

u est un nombre compris entre 1 et 20 inclus et

t est un nombre compris entre 0 et 20 inclus,

la somme (t+u) est égale ou supérieure à 3,

formules dans lesquelles le symbole A est un radical de formule :

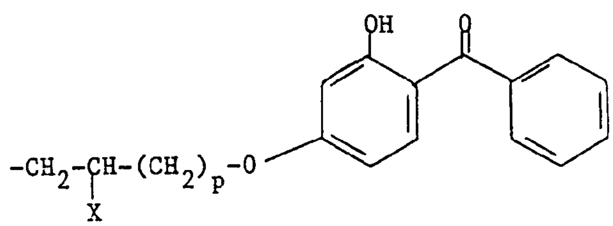 (3)

dans laquelle :

X est un atome d'hydrogène ou un radical alkyle linéaire ou ramifié en $C_1$-$C_4$;

p est un nombre entier compris entre 1 et 10 inclus.

On utilise plus particulièrement les polymères statistiques ou à blocs de formules (1) ou (2) présentant au moins l'une des caractéristiques suivantes :

- R est méthyle,
- B est méthyle,
- r est compris entre 5 et 20 inclus,
- s est compris entre 2 et 15 inclus,
- t + u est compris entre 3 et 10 inclus,
- p = 1,
- X = H ou méthyle.

Pour préparer les polymères de formules (1) et (2), on peut par exemple partir du polymère correspondant dans lequel tous les radicaux A sont des atomes d'hydrogène.

Ce polymère est dénommé par la suite polymère à SiH; les groupes SiH peuvent être présents dans la

chaîne et/ou aux extrémités de chaîne. Ces polymères à SiH sont des produits bien connus dans l'industrie des silicones et sont généralement disponibles dans le commerce.

Ils sont par exemple décrits dans les brevets américains US-A-3 220 972, US-A-3 436 366, US-A-3 697 473 et US-A-4 340 709.

Ce polymère à SiH peut donc être choisi parmi ceux de formule :

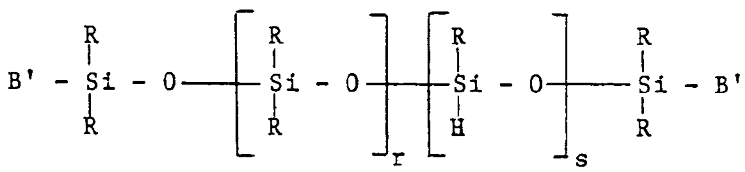

(4)

dans laquelle R, r et s ont la signification donnée ci-dessus pour la formule (1) et les radicaux B', identiques ou différents, sont choisis parmi les radicaux R et un atome d'hydrogène, et de formule :

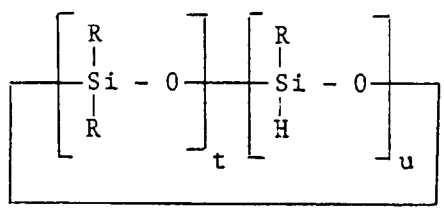

(5)

dans laquelle R, t et u ont la signification donnée ci-dessus pour la formule (2).

A l'aide de ce polymère à SiH de formule (4) ou (5), on effectue une réaction d'hydrosilylation, en présence d'une quantité catalytiquement efficace d'un catalyseur au platine, sur un dérivé organique d'hydroxy-2 benzophénone de formule :

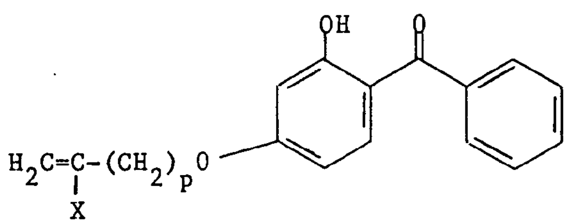

(6)

dans laquelle X et p ont les mêmes significations que dans la formule (3).

Les catalyseurs au platine utilisés pour réaliser la réaction d'hydrosilylation des polymères de formules (4) ou (5) sur le dérivé organique de formule (6) sont amplement décrits dans la littérature. On peut en particulier citer les complexes du platine et d'un produit organique décrit dans les brevets américains US-A-3 159 601, US-A-3 159 602, US-A-3 220 972 et les brevets européens EP-A-57 459, EP-A-188 978 et EP-A-190 530 et les complexes du platine et d'organopolysiloxane vinylé décrits dans les brevets américains US-A-3 419 593, US-A-3 377 432 et US-A-3 814 730.

Pour faire réagir le polymère à SiH de formule (4) ou (5) sur le dérivé de formule (6), on utilise généralement une quantité de catalyseur au platine calculée en poids de platine métal, comprise entre 5 et 600 ppm, de préférence entre 10 et 200 ppm, basée sur le poids de polymère à SiH de formule (4) ou (5).

La réaction d'hydrosilylation peut avoir lieu en masse ou au sein d'un solvant organique volatil tel que le toluène, l'heptane, le xylène, le tétrahydrofuranne et le tétrachloréthylène.

Il est généralement souhaitable de chauffer le mélange réactionnel à une température de 60 à 120°C pendant le temps nécessaire pour que la réaction soit complète. On peut ajouter goutte à goutte le polymère à SiH sur le dérivé de formule (6) en solution dans un solvant organique ou bien ajouter simultanément le polymère à SiH et le dérivé de formule (6) à une suspension de catalyseur dans le solvant organique.

On vérifie que la réaction est complète en dosant les SiH résiduels par la potasse alcoolique, puis on élimine le solvant, par exemple par distillation sous pression réduite.

L'huile brute obtenue peut être purifiée, par exemple par passage sur une colonne absorbante de silice.

Un autre objet de l'invention est constitué par les compositions cosmétiques destinées à protéger la peau et les cheveux du rayonnement UV, contenant une quantité efficace d'un diorganopolysiloxane à fonction hydroxy-2 benzophénone de formule (1) ou (2), dans un milieu cosmétiquement acceptable.

La présente invention a également pour objet un procédé de protection de la peau et des cheveux naturels ou sensibilisés vis-à-vis du rayonnement solaire, consistant à appliquer sur la peau ou les cheveux une quantité efficace d'au moins un composé de formule (1) ou (2) contenu dans un support cosmétiquement acceptable.

On entend par "cheveux sensibilisés" des cheveux ayant subi un traitement de permanente, de coloration ou de décoloration.

L'invention a également pour objet une composition cosmétique colorée ou non colorée, stabilisée à la lumière, comprenant une quantité efficace d'au moins un diorganopolysiloxane à fonction hydroxy-2 benzophénone de formule (1) ou (2) ci-dessus.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotions huileuses, alcooliques ou oléoalcooliques, d'émulsions telles qu'une crème ou un lait, de gels oléoalcooliques, alcooliques ou hydroalcooliques, de bâtonnets solides ou être conditionnée en aérosol pour former un spray ou une mousse.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (1) ou (2) est présent dans des proportions en poids comprises entre 0,25 et 3% par rapport au poids total de la composition cosmétique protectrice de l'épiderme humain.

Comme solvant de solubilisation, on peut utiliser une huile, une cire et de façon générale tout corps gras, un monoalcool ou un polyol inférieur, un benzoate d'alcools en $C_{12}$-$C_{15}$ ou leurs mélanges. Les monoalcools ou polyols plus particulièrement préférés sont l'éthanol, l'isopropanol, le propylèneglycol, l'hexylèneglycol, la glycérine et le sorbitol.

Une forme de réalisation de l'invention est une émulsion sous forme de crème ou de lait protecteurs comprenant en plus du composé de formule (1) ou (2), des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation est constituée par des lotions huileuses à base d'huiles et cires naturelles ou synthétiques, de lanoline, d'alcools gras et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylèneglycol ou la glycérine et un épaississant tel que la silice. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

Dans le cas d'une composition conditionnée en aérosol, on utilise les propulseurs classiques tels que les alcanes, les fluoroalcanes et les chlorofluoroalcanes.

La présente invention vise également les compositions cosmétiques anti-solaires contenant au moins un composé de formule (1) ou (2) et pouvant contenir d'autres filtres UV-B et/ou UV-A.

Dans ce cas, la quantité de composé de formule (1) ou (2) est comprise entre 0,5 et 10% en poids par rapport au poids total de la composition antisolaire, la quantité totale de filtres, si d'autres filtres sont présents, étant comprise entre 0,5 et 15%.

Ces compositions anti-solaires se présentent sous les formes indiquées ci-dessus pour les compositions protectrices de l'épiderme humain.

Lorsque la composition cosmétique selon l'invention est destinée à protéger des rayons UV les cheveux naturels ou sensibilisés, cette composition peut se présenter sous forme de shampooing, de lotion, mousse, gel ou émulsion à rincer, à appliquer avant ou après le shampooing, avant ou après coloration ou décoloration, avant ou après permanente, de lotion, mousse ou gel coiffants ou traitants, de lotion, mousse ou gel pour le brushing ou la mise en plis, de spray de coiffage, de laque pour cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de

mousse, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,25 à 5% en poids de composé de formule (1) ou (2).

La présente invention vise également les compositions cosmétiques renfermant un constituant sensible à la lumière et contenant au moins un composé de formule (1) ou (2) à titre d'agent de protection contre les rayons ultraviolets. Ces compositions sont constituées par des compositions capillaires telles que les laques pour cheveux, les lotions de mise en plis éventuellement traitantes ou démêlantes, les shampooings colorants, les compositions tinctoriales pour cheveux, par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière au cours du stockage.

De telles compositions contiennent 0,25 à 3% en poids de composé de formule (1) ou (2).

L'invention vise également un procédé de protection des compositions cosmétiques contre les rayons ultraviolets, consistant à incorporer à ces compositions une quantité efficace d'au moins un composé de formule (1) ou (2).

Les exemples ci-après illustrent l'invention sans en limiter la portée.

EXEMPLES DE PREPARATION

EXEMPLE 1

Préparation du polymère statistique de formule :

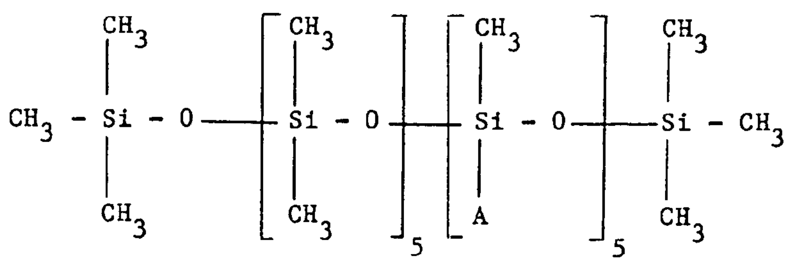

(1)

dans laquelle A est le reste de formule :

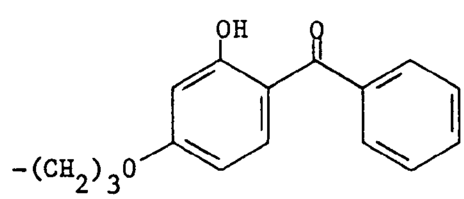

A une suspension de platine sur charbon à 5% (70 mg) dans du toluène sec (5 ml) à 90-100°C, sous azote et sous agitation, on ajoute goutte à goutte en 1 heure 30 minutes, une solution toluénique (40 ml) d'allyloxy-4 hydroxy-2 benzophénone (12,7 g, 50 meq), préparée selon le procédé décrit à l'exemple 1 du brevet US 4 278 804, et de polyméthylhydro-(45-50%) diméthylsiloxane copolymère (Petrarch Systems Inc., PS 122.5, 8,13 g, 50 meq en SiH) tout en maintenant la température entre 100 et 105°C. On laisse sous agitation et au reflux jusqu'à disparition des groupements SiH (absence de bande à 2180 $cm^{-1}$ en infrarouge), soit 10 heures. On filtre sur papier, on élimine le solvant et on lave trois fois à l'éthanol à 80%. L'huile obtenue est reprise dans le chloroforme, séchée sur sufate de sodium et filtrée sur célite pour éliminer les restes de platine colloïdal. On obtient, après évaporation du solvant, une huile épaisse jaune pâle.

Spectre UV ($CHCl_3$),

$\lambda\ max_1$ = 288 nm,

$\lambda\ max_2$ = 328 nm,

L'analyse par résonance magnétique nucléaire ($^1H$ et $^{29}Si$ RMN) indique que ce produit est bien le polymère

de formule souhaitée.

EXEMPLE 2

Huile antisolaire

| | | |
|---|---|---|
| - Composé de l'exemple 1 | | 3,5 g |
| - Huile d'amandes douces | | 3,0 g |
| - Parfum | | 1,2 g |
| - Benzoate d'alcools $C_{12}/C_{15}$ ("FINSOLV TN" vendu par la Société WITCO) | qsp | 100 g |

EXEMPLE 3

Emulsion eau-dans-l'huile antisolaire

| | | |
|---|---|---|
| - Composé de l'exemple 1 | | 3,0 g |
| - Mélange de diorganopolysiloxanes et d'émulsionnants cétyldiméthicone copolyol/cétyldiméthicone/polyglycéryl 3-oléate/hexyl laurate/ ("ABIL WS 08" vendu par la Société GOLDSCHMIDT) | | 5,0 g |
| - Benzoate d'alcools $C_{12}/C_{15}$ ("FINSOLV TN" vendu par la Société WITCO) | | 12,0 g |
| - Vaseline | | 2,0 g |
| - Cire d'abeilles | | 2,5 g |
| - Glycérine | | 2,0 g |
| - Chlorure de sodium | | 2,0 g |
| - Conservateur | | 0,2 g |
| - Parfum | | 0,6 g |
| - Eau déminéralisée | qsp | 100 g |

EXEMPLE 4

Emulsion huile-dans-eau protectrice de l'épiderme humain

| | | |
|---|---|---|
| - Composé de l'exemple 1 | | 1,5 g |
| - Mélange d'alcool cétylstéarylique et cétylstéarylique oxyéthyléné à 33 moles OE ("SINNOWAX AO" vendu par la Société HENKEL) | | 7,0 g |
| - Monostéarate de glycérol | | 2,0 g |
| - Propylèneglycol | | 10,0 g |
| - Alcool cétylique | | 1,3 g |
| - Benzoate d'alcools $C_{12}/C_{15}$ ("FINSOLV TN" vendu par la Société WITCO) | | 15,0 g |
| - Conservateur | | 0,2 g |
| - Parfum | | 0,6 g |
| - Eau déminéralisée | qsp | 100 g |

L'émulsion de l'exemple 4 est préparée de la façon suivante :

on chauffe les corps gras et les émulsionnants vers 80-85°C; on ajoute le composé de l'exemple 1. Par ailleurs on chauffe à 80-85°C l'eau contenant les composés hydrosolubles et on ajoute la phase grasse à la phase aqueuse. Après 10 minutes d'agitation vive, on laisse refroidir sous agitation modérée et vers 40°C, on ajoute le conservateur et le parfum.

L'émulsion de l'exemple 3 est préparée de la même façon sauf que l'on ajoute la phase aqueuse à la phase grasse.

EXEMPLE 5

Huile protectrice des cheveux

| | | |
|---|---|---|
| - Composé de l'exemple 1 | | 1 g |
| - Alcool oléique | | 19,5 g |
| - Hexylène glycol | | 0,5 g |
| - Huile de colza | qsp | 100 g |

Cette huile, d'aspect opalescent, est appliquée sur cheveux séchés auxquels elle apporte de la brillance et de la douceur tout en préservant leur couleur et leurs propriétés mécaniques lorsqu'ils sont exposés à la lumière naturelle.

EXEMPLE 6

Mousse capillaire protectrice

| | | |
|---|---|---|
| - Composé de l'exemple 1 | | 1 g |
| - Nonylphénol oxyéthylèné à 10 moles d'oxyde d'éthylène vendu sous le nom de "MONTANOX 1030" par la Société SEPPIC | | 10 g |
| - Chlorure de diméthyl dialkyl (suif) ammonium | | 0,1 g |
| - Eau | qsp | 100 g |

Cette composition est pressurisée dans un dispositif aérosol dans les proportions de 90 g de composition pour 10 g de propulseur constitué par du butane 3,2N.

Cette composition donne lieu à la formation d'une mousse que l'on applique sur cheveux lavés et essorés. Après quelques minutes de pose, les cheveux sont rincés. Ils se démêlent facilement et sont doux après séchage.

EXEMPLE 7

Crème capillaire protectrice

| | | |
|---|---|---|
| - Composé de l'exemple 1 | | 0,5 g |
| - Alcool cétylstéarylique oxyéthylèné à 33 moles d'oxyde d'éthylène | | 4 g |
| - Alcool cétylique | | 2 g |
| - Alcool stéarylique | | 2 g |
| - Ether de cellulose cationique vendu sous la dénomination "JR400" par la Société UNION CARBIDE | | 0,5 g |
| - Eau | qsp | 100 g |

Cette émulsion est préparée comme dans l'exemple 4.

Appliquée sur cheveux mouillés, elle apporte une facilité de démêlage. Les cheveux séchés sont doux et sont protégés du soleil.

**Revendications**

1. Utilisation en cosmétique de diorganopolysiloxanes à fonction hydroxy-2 benzophénone choisis parmi ceux de formule :

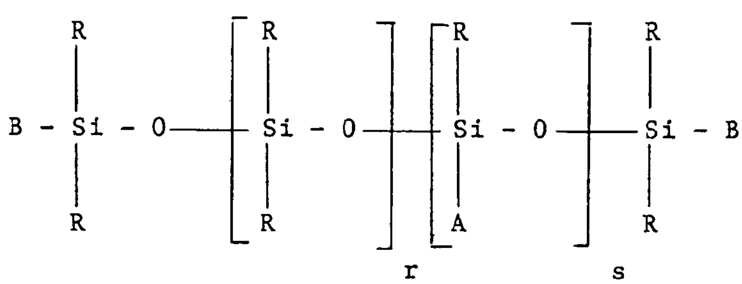 (1)

dans laquelle les symboles :

R, identiques ou différents, sont choisis parmi les radicaux alkyle linéaires ou ramifiés en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

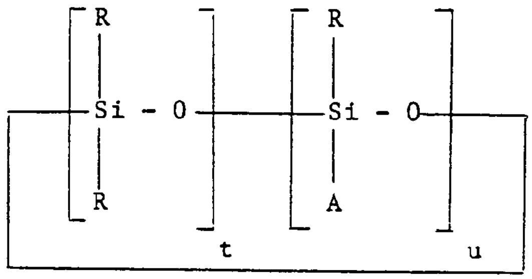

(2)

dans laquelle

R a la même signification qu'à la formule (1),

u est un nombre compris entre 1 et 20 inclus,

t est un nombre compris entre 0 et 20 inclus,

la somme (t+u) est égale ou supérieure à 3,

formules dans lesquelles le symbole A est un radical de formule :

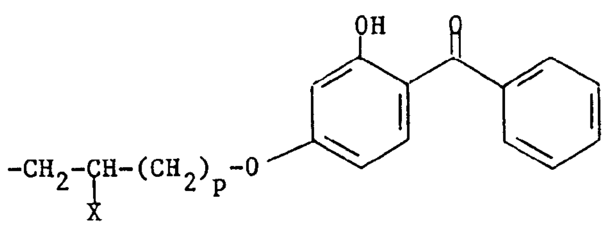

(3)

dans laquelle :

X est un atome d'hydrogène ou un radical alkyle linéaire ou ramifié en $C_1$-$C_4$,

p est un nombre entier compris entre 1 et 10 inclus.

2. Utilisation en cosmétique d'un polymère statistique ou à blocs de formule (1) ou (2) selon la revendication 1, présentant au moins l'une des caractéristiques suivantes :

R est méthyle,

B est méthyle,

r est compris entre 5 et 20 inclus,

s est compris entre 2 et 15 inclus,

t + u est compris entre 3 et 10 inclus,

p = 1,

X est H ou méthyle.

3. Utilisation en cosmétique à titre d'agents filtrant le rayonnement UV de longueurs d'onde comprises entre 280 et 360 nm, de diorganopolysiloxanes à fonction hydroxy-2 benzophénone choisis parmi ceux de formule :

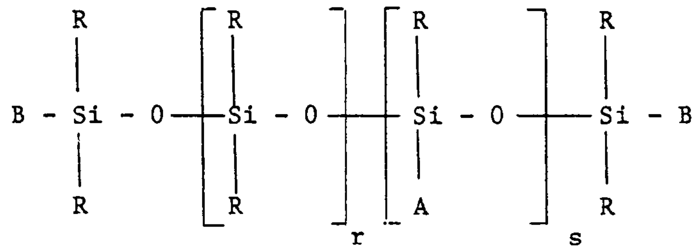 (1)

dans laquelle les symboles :

R, identiques ou différents, sont choisis parmi les radicaux alkyle linéaires ou ramifiés en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

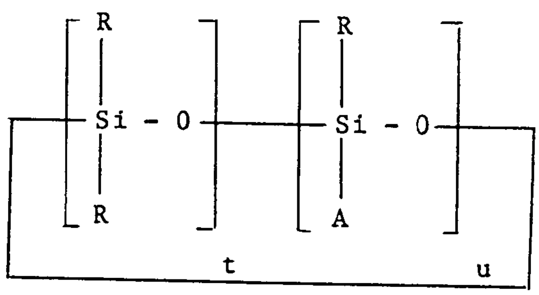 (2)

dans laquelle

R a la même signification qu'à la formule (1),

u est un nombre compris entre 1 et 20 inclus,

t est un nombre compris entre 0 et 20 inclus,

la somme (t+u) est égale ou supérieure à 3,

formules dans lesquelles le symbole A est un radical de formule :

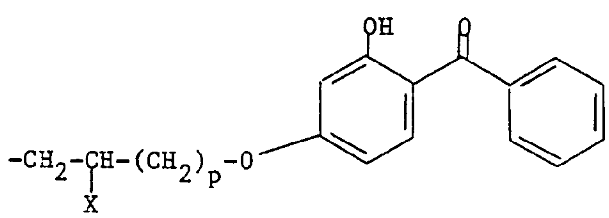 (3)

dans laquelle :

X est un atome d'hydrogène ou un radical alkyle linéaire ou ramifié en $C_1$-$C_4$,

p est un nombre entier compris entre 1 et 10 inclus.

**4.** Utilisation en cosmétique à titre d'agents filtrant le rayonnement UV de longueurs d'onde comprises entre 280 et 360 nm, de diorganopolysiloxanes statistiques ou à blocs de formule (1) ou (2) selon la revendication 3, présentant au moins l'une des caractéristiques suivantes :

R est méthyle

B est méthyle

r est compris entre 5 et 20 inclus,

s est compris entre 2 et 15 inclus,

t + u est compris entre 3 et 10 inclus,

p = 1,
X est H ou méthyle.

**5.** Utilisation en cosmétique à titre d'agent filtrant le rayonnement UV de longueurs d'onde comprises entre 280 et 360 nm, d'un polydiméthylsiloxane à greffons allyloxy-4 hydroxy-2 benzophénone de formule (1) selon la revendication 1, dans laquelle R et B désignent méthyle, r est égal à 5 et s est égal à 5.

**6.** Composition cosmétique, caractérisée par le fait qu'elle comprend, dans un support cosmétiquement acceptable, une quantité efficace d'au moins un diorganopolysiloxane à fonction hydroxy-2 benzophénone choisi parmi ceux de formule :

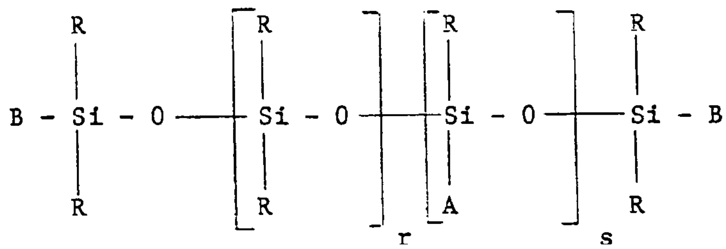

(1)

dans laquelle les symboles :

R, identiques ou différents, sont choisis parmi les radicaux alkyle linéaires ou ramifiés en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

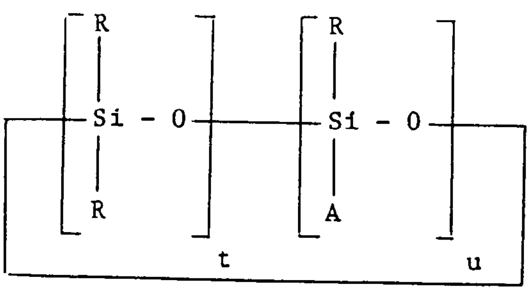

(2)

dans laquelle

R a la même signification qu'à la formule (1),

u est un nombre compris entre 1 et 20 inclus,

t est un nombre compris entre 0 et 20 inclus,

la somme (t+u) est égale ou supérieure à 3,

formules dans lesquelles le symbole A est un radical de formule :

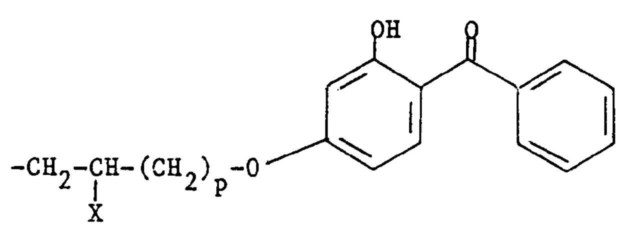

(3)

dans laquelle :

X est un atome d'hydrogène ou un radical alkyle linéaire ou ramifié en $C_1$-$C_4$,

p est un nombre entier compris entre 1 et 10 inclus.

7. Composition cosmétique selon la revendication 6, caractérisée par le fait qu'elle comprend un diorganopolysiloxane à fonction hydroxy-2 benzophénone de formule (1) ou (2), statistique ou à blocs, présentant au moins l'une des caractéristiques suivantes : R est méthyle, B est méthyle, r est compris entre 5 et 20 inclus, s est compris entre 2 et 15 inclus, t + u est compris entre 3 et 10 inclus, p est égal à 1, X est H ou méthyle.

8. Composition cosmétique selon la revendication 6 ou 7, caractérisée par le fait qu'elle comprend un polydiméthylsiloxane à greffons allyloxy-4 hydroxy-2 benzophénone de formule (1) dans laquelle R et B désignent méthyle, r est égal à 5 et s est égal à 5.

9. Composition cosmétique selon l'une quelconque des revendications 6 à 8, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, benzoates d'alcools en $C_{12}$-$C_{15}$, propulseurs, colorants et pigments.

10. Composition cosmétique selon l'une quelconque des revendications 4 à 9, caractérisée par le fait qu'elle se présente sous forme de lotion huileuse, alcoolique ou oléoalcoolique, d'émulsion, de gel oléoalcoolique, alcoolique ou hydroalcoolique, de bâtonnet solide, de spray ou de mousse.

11. Composition cosmétique selon l'une quelconque des revendications 6 à 10, caractérisée par le fait qu'elle constitue une composition protectrice de l'épiderme humain et contient 0,25 à 3% en poids de diorganopolysiloxane de formule (1) ou (2).

12. Composition cosmétique selon l'une quelconque des revendications 6 à 10, se présentant sous forme de composition anti-solaire, caractérisée par le fait qu'elle contient 0,5 à 10% en poids de diorganopolysiloxane de formule (1) ou (2).

13. Composition cosmétique anti-solaire selon la revendication 12, caractérisée par le fait qu'elle contient en outre un agent filtrant les rayons UV-B et/ou UV-A.

14. Composition cosmétique selon l'une quelconque des revendications 6 à 9, destinée à être appliquée sur les cheveux, caractérisée par le fait qu'elle se présente sous forme de shampooing, lotion, gel, mousse ou émulsion à rincer, à appliquer avant ou après le shampooing, avant ou après coloration ou décoloration, avant ou après permanente, lotion, mousse ou gel coiffants ou traitants, lotion, mousse ou gel pour le brushing ou la mise en plis, spray de coiffage, laque pour cheveux, et comprend 0,25 à 5% en poids de diorganopolysiloxane de formule (1) ou (2).

15. Composition cosmétique selon l'une quelconque des revendications 6 à 9, se présentant sous forme d'une composition cosmétique colorée ou non, renfermant un constituant sensible à la lumière, caractérisée par le fait qu'elle est constituée par une composition capillaire, un produit de maquillage ou une composition pour les soins ou le traitement de la peau, comprenant 0,25 à 3% en poids de diorganopolysiloxane de formule (1) ou (2).

16. Procédé de protection de la peau et des cheveux naturels ou sensibilisés contre le rayonnement ultraviolet, caractérisé par le fait qu'il consiste à appliquer sur la peau ou les cheveux une quantité efficace d'une composition cosmétique contenant au moins un diorganopolysiloxane à fonction hydroxy-2 benzophénone de formule (1) ou (2) définie dans l'une quelconque des revendications 3 à 5.

17. Procédé de protection d'une composition cosmétique contre les rayons ultraviolets, caractérisé par le fait qu'il consiste à incorporer à cette composition une quantité efficace d'au moins un diorganopolysiloxane à fonction hydroxy-2 benzophénone de formule (1) ou (2) définie dans l'une quelconque des revendications 3 à 5.

**Patentansprüche**

1. Verwendung von Diorganopolysiloxanen mit einer 2-Hydroxybenzophenon-Gruppierung in der Kosmetik, ausgewählt aus solchen der Formel:

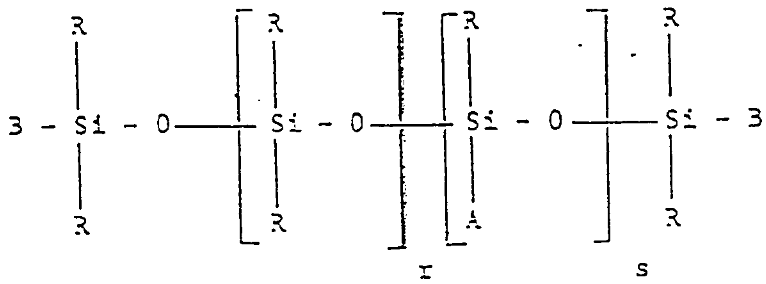

(1)

worin die Symbole bedeuten:

R ist identisch oder verschieden und ausgewählt aus den geradkettigen oder verzweigten $C_1$-$C_{10}$-Alkylradikalen, Phenyl und 3,3,3-Trifluor-propyl, wobei mindestens 80 % der Zahl der R-Radikale Methylradikale sind,

B ist identisch oder verschieden, und ausgewählt aus den Radikalen R und dem Radikal A,

r ist eine Zahl ausgewählt zwischen 0 und einschließlich 200,

s ist eine Zahl ausgewählt zwischen 0 und einschließlich 50, und wenn s 0 ist, ist mindestens eines der beiden Symbole B A, und solchen der Formel:

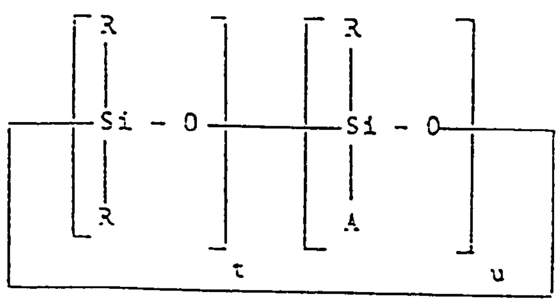

(2)

worin R die gleiche Bedeutung wie in Formel (1) besitzt,
u eine Zahl zwischen 1 und einschließlich 20 ist, und
t eine Zahl zwischen 0 und einschließlich 20 ist,
die Summe (t + u) gleich oder größer als 3 ist,
und in denen das Symbol A ein Radikal der Formel ist:

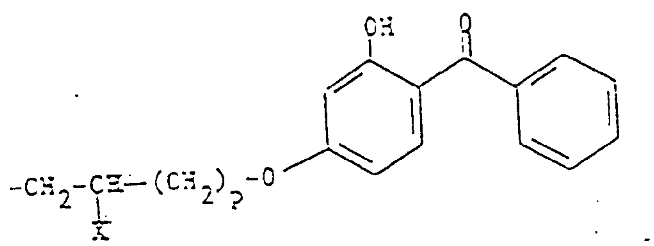

(3)

worin X ein Wasserstoffatom oder ein geradkettiges oder verzweigtes $C_1$-$C_4$-Alkylradikal ist;
p eine ganze Zahl zwischen 1 und einschließlich 10 ist.

2. Verwendung eines statistischen Polymeren oder Blockpolymeren der Formel (1) oder (2) gemäß Anspruch 1 in der Kosmetik, dadurch gekennzeichnet, daß sie mindestens eines der folgenden Merkmale aufweisen:
   - R ist Methyl
   - B ist Methyl
   - r liegt zwischen 5 und einschließlich 20,
   - s liegt zwischen 2 und einschließlich 15,
   - t + u liegt zwischen 3 und einschließlich 10,
   - p = 1
   - X = H oder Methyl.

3. Verwendung eines Diorganopolysiloxans mit einer 2-Hydroxy-benzophenon-Gruppierung in der Kosmetik als Filtermittel für UV-Strahlung im Wellenlängenbereich zwischen 280 und 360 nm ausgewählt aus solchen der Formel

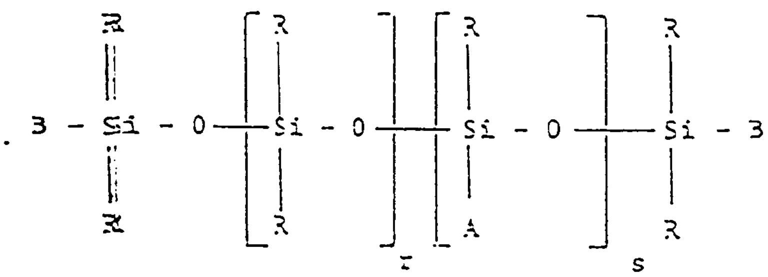

(1)

worin die Symbole bedeuten:

R ist identisch oder verschieden und ausgewählt aus den geradkettigen oder verzweigten $C_1$-$C_{10}$-Alkylradikalen, Phenyl oder 3,3,3-Trifluor-propyl, wobei mindestens 80 % der Zahl der R-Radikale Methylradikale sind,

B ist identisch oder verschieden, und ausgewählt aus den Radikalen R und dem Radikal A,

r ist eine Zahl ausgewählt zwischen 0 und einschließlich 200,

s ist eine Zahl ausgewählt zwischen 0 und einschließlich 50, und wenn s 0 ist, ist mindestens eines der beiden Symbole B A, und solchen der Formel

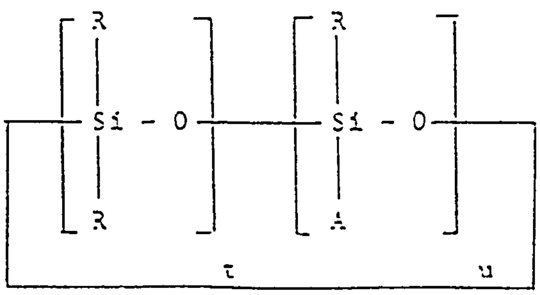

(2)

worin R die gleiche Bedeutung wie in Formel (1) besitzt,
u eine Zahl zwischen 1 und einschließlich 20 ist, und
t eine Zahl zwischen 0 und einschließlich 20 ist,
die Summe (t + u) gleich oder größer als 3 ist,
und in denen das Symbol A ein Radikal der Formel ist:

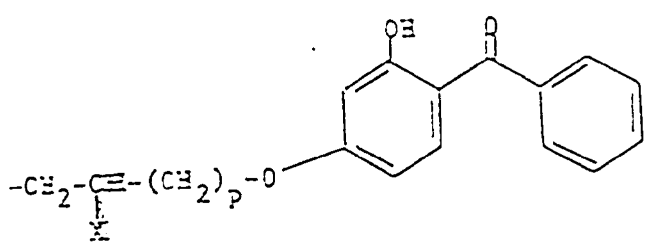

(3)

worin X ein Wasserstoffatom oder ein geradkettiges oder verzweigtes $C_1$-$C_4$-Alkylradikal ist;
p eine ganze Zahl zwischen 1 und einschließlich 10 ist.

4. Verwendung von statistischen oder Block-Diorganopolysiloxanen der Formel (1) oder (2) in der Kosmetik als Filter für UV-Strahlung der Wellenlänge zwischen 280 und 360 nm, dadurch gekennzeichnet, daß sie mindestens eines der folgenden Merkmale aufweisen:
- R ist Methyl
- B ist Methyl
- r liegt zwischen 5 und einschließlich 20,

- s liegt zwischen 2 und einschließlich 15,
- t + u liegt zwischen 3 und einschließlich 10,
- p = 1
- X = H oder Methyl.

5. Verwendung eines Diorganopolysiloxans mit Pfropfen aus 4-Allyloxy-2-hydroxy-benzophenon der Formel (1) gemäß Anspruch 1 in der Kosmetik, dadurch gekennzeichnet, daß R und B Methyl bedeuten, r = 5 ist, und s = 5 ist.

6. Kosmetische Zusammensetzung, dadurch gekennzeichnet, daß sie in einem kosmetisch annehmbaren Träger eine wirksame Menge mindestens eines Diorganopolysiloxans mit 2-Hydroxybenzophenon-Gruppierung enthält, ausgewählt aus solchen der Formel:

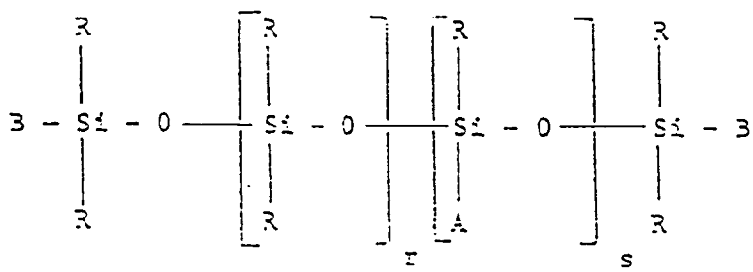

(1)

worin die Symbole bedeuten:

R ist identisch oder verschieden und ausgewählt aus den geradkettigen oder verzweigten $C_1$-$C_{10}$-Alkylradikalen, Phenyl und 3,3,3-Trifluor-propyl, wobei mindestens 80 % der Zahl der R-Radikale Methylradikale sind,

B ist identisch oder verschieden, und ausgewählt aus den Radikalen R und dem Radikal A,

r ist eine Zahl ausgewählt zwischen 0 und einschließlich 200,

s ist eine Zahl ausgewählt zwischen 0 und einschließlich 50, und wenn s 0 ist, ist mindestens eines der beiden Symbole B A, und solchen der Formel

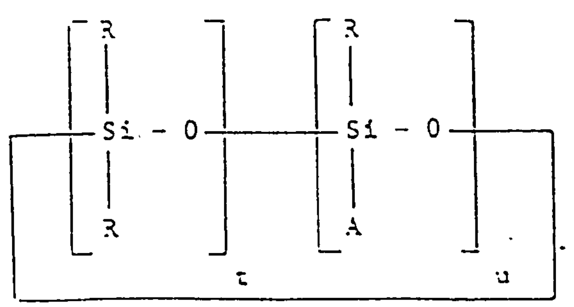

(2)

worin R die gleiche Bedeutung wie in Formel (1) besitzt,
u eine Zahl zwischen 1 und einschließlich 20 ist, und
t eine Zahl zwischen 0 und einschließlich 20 ist,
die Summe (t + u) gleich oder größer als 3 ist,
und in denen das Symbol A ein Radikal der Formel ist:

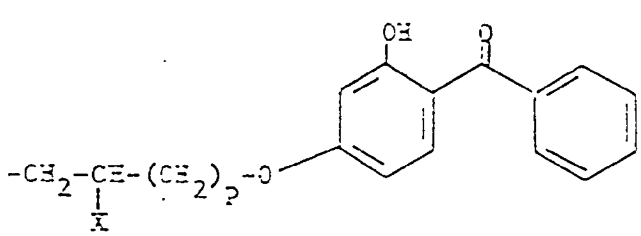

(3)

worin X ein Wasserstoffatom oder ein geradkettiges oder verzweigtes $C_1$-$C_4$-Alkylradikal ist; p eine ganze Zahl zwischen 1 und einschließlich 10 ist.

7. Kosmetische Zusammensetzung nach Anspruch 6, dadurch gekennzeichnet, daß sie ein Diorganopolysiloxan mit 2-Hydroxybenzophenon-Gruppierung der Formel (1) oder (2) als statistisches oder Block-Polymeres enthält, das mindestens eines der folgenden Merkmale besitzt:
   - R ist Methyl
   - B ist Methyl
   - r liegt zwischen 5 und einschließlich 20,
   - s liegt zwischen 2 und einschließlich 15,
   - t + u liegt zwischen 3 und einschließlich 10,
   - p = 1
   - X = H oder Methyl.

8. Kosmetische Zusammensetzung nach Anspruch 6 oder 7, dadurch gekennzeichnet, daß sie ein Polydimethylsiloxan mit Pfropfen aus 4-Allyloxy-2-hydroxy-benzophenon der Formel (1) enthält, worin R und B Methyl bedeuten, r = 5 und s = 5.

9. Kosmetische Zusammensetzung gemäß einem der Ansprüche 6 bis 8, dadurch gekennzeichnet, daß sie außerdem kosmetische Additive enthält, ausgewählt unter Verdickungsmitteln, Weichmachern, Feuchthaltemitteln, oberflächenaktive Mitteln, Konservierungsmitteln, Antischaummitteln, Parfüms, Ölen, Wachsen, Lanolin, niederen Monoalkoholen und Polyolen, Benzoaten von $C_{12}$-$C_{15}$-Alkoholen, Treibmitteln, Farbstoffen und Pigmenten.

10. Kosmetische Zusammensetzung nach einem der Ansprüche 4 bis 9, dadurch gekennzeichnet, daß sie in Form einer öligen, alkoholischen oder oleoalkoholischen Lotion, einer Emulsion, eines oleoalkoholischen, alkoholischen oder hydroalkoholischen Gels, als fester Stift, als Spray oder Schaum vorliegt.

11. Kosmetische Zusammensetzung nach einem der Ansprüche 6 bis 10, dadurch gekennzeichnet, daß sie eine Zusammensetzung zum Schutz der menschlichen Epidermis ist und 0.25 bis 3 Gew.% Diorganopolysiloxan der Formel (1) oder (2) enthält.

12. Kosmetische Zusammensetzung nach einem der Ansprüche 6 bis 10 in Form einer Sonnenschutzmittel-Zusammensetzung, dadurch gekennzeichnet, daß sie 0.5 bis 10 Gew.-% Diorganopolysiloxan der Formel (1) oder (2) enthält.

13. Kosmetische Sonnenschutz-Zusammensetzung gemäß Anspruch 12, dadurch gekennzeichnet, daß sie außerdem einen Filter für UV-B- und/oder UV-A-Strahlen enthält.

14. Kosmetische Zusammensetzung nach einem der Ansprüche 6 bis 9, bestimmt zur Applikation auf den Haaren, dadurch gekennzeichnet, daß sie in Form eines Shampoos, einer Lotion, eines Gels, eines Schaums oder einer Spülemulsion, zur Applikation vor oder nach dem Shampoonieren, vor oder nach dem Färben oder Entfärben, vor oder nach der Dauerwellung, einer Frisier- oder Formgebungs-Lotion, -Schaums oder -Gels, einer Fön- oder Wasserwell-Lotion, Schaum- oder Gel, eines Frisiersprays, eines Haarlackes, vorliegt, und 0.25 bis 5 Gew.-% Diorganopolysiloxan der Formel (1) oder (2) enthält.

15. Kosmetische Zusammensetzung nach einem der Ansprüche 6 bis 9 in Form einer gefärbten oder nichtgefärbten kosmetischen Zusammensetzung, die einen gegenüber Licht empfindlichen Bestandteil enthält, dadurch gekennzeichnet, daß sie eine Haarbehandlungs-Zusammensetzung ist, ein Schminkprodukt oder eine Zusammensetzung zur Pflege und Behandlung der Haut, und 0.25 bis 3 Gew.-% Diorganopolysiloxan der Formel (1) oder (2) enthält.

16. Verfahren zum Schutz der Haut und von natürlichen oder sensibilisierten Haaren gegen ultraviolette Strahlen, dadurch gekennzeichnet, daß man auf die Haut oder die Haare eine wirksame Menge einer kosmetischen Zusammensetzung aufbringt, die mindestens ein Diorganopolysiloxan mit einer 2-Hydroxybenzophenon-Gruppierung der Formel (1) oder (2), wie sie in einem der Ansprüche 3 bis 5 definiert sind, enthält.

17. Verfahren zum Schutz einer kosmetischen Zusammensetzung gegen ultraviolette Strahlung, dadurch gekennzeichnet, daß man in diese Zusammensetzung eine wirksame Menge von mindestens einem Diorganopolysiloxan mit 2-Hydroxy-benzophenon-Gruppierung der Formel (1) oder (2), wie sie in einem der Ansprüche 3 bis 5 definiert sind, einbringt.

## Claims

1. Use in cosmetics of diorganopolysiloxanes containing a 2-hydroxybenzophenone functional group, which are chosen from those of formula:

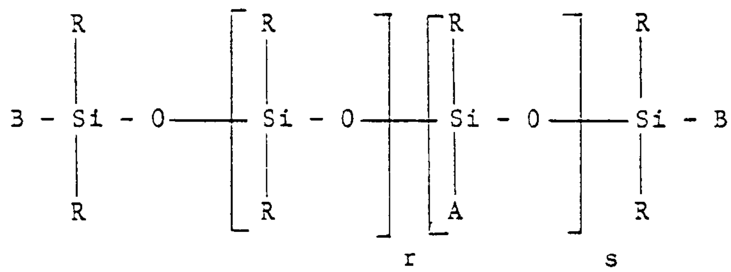

(1)

in which the symbols:

R, which are identical or different, are chosen from linear or branched $C_1$-$C_{10}$ alkyl, phenyl and 3,3,3-trifluoropropyl radicals, at least 80% of the number of the radicals R being methyl radicals,

B, which are identical or different, are chosen from the radicals R and the radical A,

r is a number chosen between 0 and 200 inclusive,

s is a number chosen between 0 and 50 inclusive

and if s is 0, at least one of the two symbols B denotes A,

and those of formula:

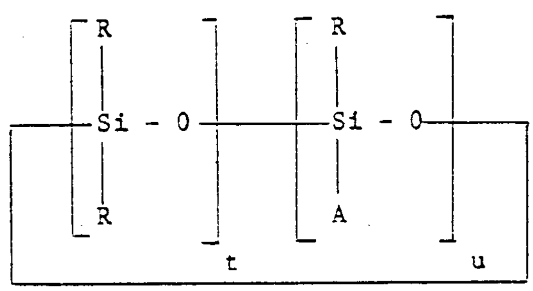

(2)

in which

R has the same meaning as in formula (1),

u is a number between 1 and 20 inclusive,

t is a number between 0 and 20 inclusive,

the sum (t+u) is equal to or greater than 3,

in which formulae the symbol A is a radical of formula:

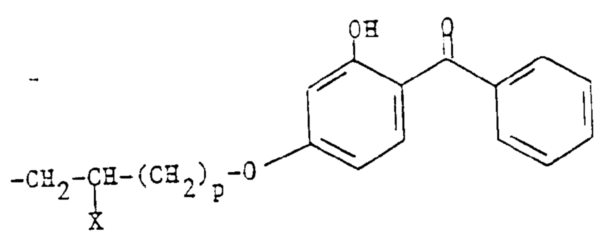

(3)

in which:

X is a hydrogen atom or a linear or branched $C_1$-$C_4$ alkyl radical, and
p is an integer between 1 and 10 inclusive.

**2.** Use in cosmetics of a random or block polymer of formula (1) or (2) according to Claim 1, exhibiting at least one of the following characteristics:

R is methyl,
B is methyl,
r is between 5 and 20 inclusive,
s is between 2 and 15 inclusive,
t + u is between 3 and 10 inclusive,
p = 1,
X is H or methyl.

**3.** Use in cosmetics, as agents screening out the UV radiation of wavelengths between 280 and 360 nm, of diorganopolysiloxanes containing a 2-hydroxybenzophenone functional group, which are chosen from those of formula:

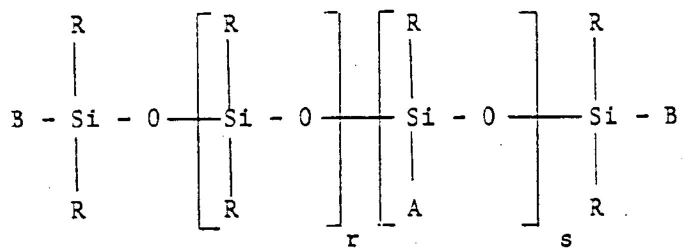
(1)

in which the symbols:

R, which are identical or different, are chosen from linear or branched $C_1$-$C_{10}$ alkyl, phenyl and 3,3,3-trifluoropropyl radicals, at least 80% of the number of the radicals R being methyl radicals,
B, which are identical or different, are chosen from the radicals R and the radical A,
r is a number chosen between 0 and 200 inclusive,
s is a number chosen between 0 and 50 inclusive
and if s is 0, at least one of the two symbols B denotes A,
and those of formula:

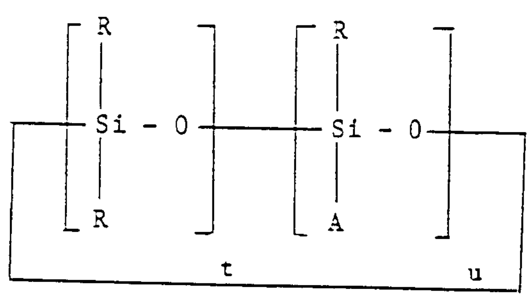
(2)

in which

R has the same meaning as in formula (1),
u is a number between 1 and 20 inclusive,
t is a number between 0 and 20 inclusive,
the sum (t+u) is equal to or greater than 3,
in which formulae the symbol A is a radical of formula:

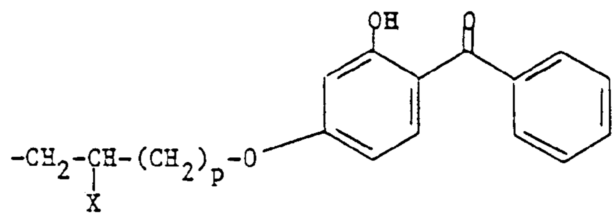

(3)

in which:

X is a hydrogen atom or a linear or branched $C_1$-$C_4$ alkyl radical, and

p is an integer between 1 and 10 inclusive.

4. Use in cosmetics, as agents screening out the UV radiation of wavelengths between 280 and 360 nm, of random or block diorganopolysiloxanes of formula (1) or (2) according to Claim 3, exhibiting at least one of the following characteristics:

R is methyl

B is methyl

r is between 5 and 20 inclusive,

s is between 2 and 15 inclusive,

t + u is between 3 and 10 inclusive,

p = 1,

X is H or methyl.

5. Use in cosmetics, as an agent screening out the UV radiation of wavelengths between 280 and 360 nm, of a polydimethylsiloxane with 4-allyloxy-2-hydroxybenzophenone grafts of formula (1) according to Claim 1, in which R and B denote methyl, r is equal to 5 and s is equal to 5.

6. Cosmetic composition, characterized in that it comprises, in a cosmetically acceptable substrate, an effective quantity of at least one diorganopolysiloxane containing a 2-hydroxybenzophenone functional group, chosen from those of formula:

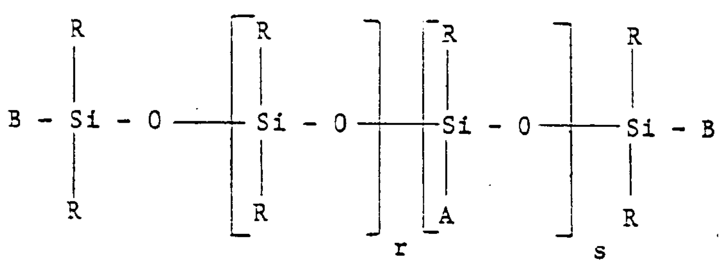

(1)

in which the symbols:

R, which are identical or different, are chosen from linear or branched $C_1$-$C_{10}$ alkyl, phenyl and 3,3,3-trifluoropropyl radicals, at least 80% of the number of the radicals R being methyl radicals,

B, which are identical or different, are chosen from the radicals R and the radical A,

r is a number chosen between 0 and 200 inclusive,

s is a number chosen between 0 and 50 inclusive and if s is 0, at least one of the two symbols B denotes A,

and those of formula:

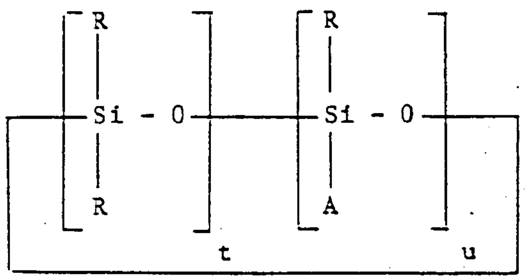

(2)

in which

R has the same meaning as in formula (1),
u is a number between 1 and 20 inclusive,
t is a number between 0 and 20 inclusive,
the sum (t+u) is equal to or greater than 3,

in which formulae the symbol A is a radical of formula:

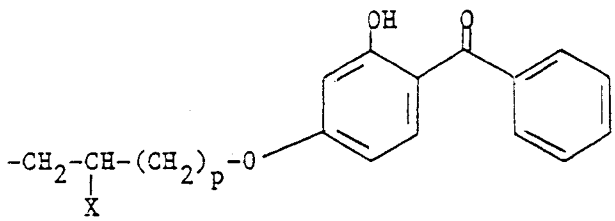

(3)

in which:

X is a hydrogen atom or a linear or branched $C_1$-$C_4$ alkyl radical, and
p is an integer between 1 and 10 inclusive.

**7.** Cosmetic composition according to Claim 6, characterized in that it comprises a diorganopolysiloxane containing a 2-hydroxybenzophenone functional group of formula (1) or (2), random or containing blocks, exhibiting at least one of the following characteristics: R is methyl, B is methyl, r is between 5 and 20 inclusive, s is between 2 and 15 inclusive, t + u is between 3 and 10 inclusive, p is equal to 1, X is H or methyl.

**8.** Cosmetic composition according to Claim 6 or 7, characterized in that it comprises a polydimethylsiloxane containing 4-allyloxy-2-hydroxybenzophenone grafts of formula (1) in which R and B denote methyl, r is equal to 5 and s is equal to 5.

**9.** Cosmetic composition according to any one of Claims 6 to 8, characterized in that it additionally contains cosmetic adjuvants chosen from thickeners, softeners, humectants, surfactants, preserving agents, antifoams, perfumes, oils, waxes, lanolin, lower monoalcohols and polyols, benzoates of $C_{12}$-$C_{15}$ alcohols, propellants, colorants and pigments.

**10.** Cosmetic composition according to any one of Claims 4 to 9, characterized in that it is in the form of an oily, alcoholic or oleoalcoholic lotion, an emulsion, an oleoalcoholic, alcoholic or hydroalcoholic gel, a solid stick, a spray or a foam.

**11.** Cosmetic composition according to any one of Claims 6 to 10, characterized in that it constitutes a composition for protecting human skin and contains 0.25 to 3% by weight of diorganopolysiloxane of formula (1) or (2).

**12.** Cosmetic composition according to any one of Claims 6 to 10, which is in the form of a sunscreen composition, characterized in that it contains 0.5 to 10% by weight of diorganopolysiloxane of formula (1) of (2).

**13.** Sunscreen cosmetic composition according to Claim 12, characterized in that it additionally contains an

agent screening out UV-B and/or UV-A rays.

**14.** Cosmetic composition according to any one of Claims 6 to 9, intended to be applied to hair, characterized in that it is in the form of a shampoo, lotion, gel, foam or emulsion for rinsing, for applying before or after shampooing, before or after dyeing or bleaching or before or after permanent waving, a hair styling or conditioning lotion, foam or gel, a lotion, foam or gel for blow drying or hair setting, a hair styling spray or a hair lacquer, and comprises 0.25 to 5% by weight of diorganopolysiloxane of formula (1) or (2).

**15.** Cosmetic composition according to any one of Claims 6 to 9, which is in the form of a coloured or uncoloured cosmetic composition, including a light-sensitive constituent, characterized in that it consists of a hair-care composition, a makeup product or a composition for skin care or treatment, comprising 0.25 to 3% by weight of diorganopolysiloxane of formula (1) or (2).

**16.** Process for protecting the skin and natural or sensitized hair against ultraviolet radiation, characterized in that it consists in applying to the skin or hair an effective quantity of a cosmetic composition containing at least one diorganopolysiloxane containing a 2-hydroxybenzophenone functional group of formula (1) or (2) defined in any one of Claims 3 to 5.

**17.** Process for protecting a cosmetic composition against ultraviolet rays, characterized in that it consists in incorporating in this composition an effective quantity of at least one diorganopolysiloxane containing a 2-hydroxybenzophenone functional group of formula (1) or (2) defined in any one of Claims 3 to 5.